# EUROPEAN PATENT APPLICATION

(11) **EP 1 938 762 A1**
(43) Date of publication of application: **02.07.2008**
(21) Application number: 07116911.4
(22) Date of filing: 21.09.2007
(51) Int. Cl.: A61B 17/04

(54) **Surgical suture device**

(30) Priority: 29.12.2006 IT TO20060933
(71) Applicant: Vassoney, Pierfrancesco, 10129 Torino (IT); Mandrile, Roberto, 12011 Borgo San Dalmazzo (CN) (IT); Trabucco, Francesco, 12038 Savigliano (CN) (IT)
(72) Inventor: Vassoney, Pierfrancesco, 10129 Torino (IT)
(74) Representative: Notaro, Giancarlo

(57) **Abstract**

Described herein is a surgical suture device comprising a flexible oblong body (2) that can be bent in a loop configuration, in which an engagement portion (3) and a clamping element (4) made in said body are brought into mutual engagement for fixing the loop configuration, said engagement portion presenting an outer surface provided with at least one projection (3), designed to contrast against an arrest element (7) of said clamping element (4), said device being characterized in that said clamping element comprises a casing (5) that encloses said arrest element (7).

## Description

### TEXT OF THE DESCRIPTION

The present invention relates to a re-absorbable or non-reabsorbable surgical device that can be used for suturing in arthroscopy, endoscopy, or in open surgery.

The operation of suture is a surgical technique that enables two margins of a tissue to be brought up to one another and to be kept in said position for a period of time sufficiently long to enable cicatrization of the two margins. The surgical threads used for said purpose are applied through portions of the two margins and fastened generally via surgical knots in configurations that keep the margins in contact with one another.

The resistance of the fastening of the threads constitutes a fundamental characteristic. In particular, in the framework of the technique in question there exist a wide range of techniques for the production of surgical knots that will guarantee a stable fastening of the threads.

Said techniques generally envisage making a single straight knot (intertwined once), or else a double straight knot, (intertwined twice), followed by an additional knot, referred to as "plane overknot". Usually, there is moreover made a third knot for final fixing, but in the case of some types of threads the fixing knots that are necessary may be as many as five.

The techniques referred to above entail elaborate manual operations on the part of the surgeon. In particular, in surgical interventions supported by endoscopy the execution of the knots is further complicated in that the surgeon controls the area of intervention indirectly via a screen that deprives him of the sensation of depth. This entails times for making the suture that are longer than the time usually necessary for sutures similar in operations in open surgery and renders execution itself of the suture an extremely delicate step of the intervention.

In the framework of the technique in question, there have been developed solutions that envisage the use of suture threads that are fastened without a knot.

For example, the patent No. DE4302895 describes a suture thread that has on its outer surface a plurality of projections distributed throughout the length of the thread, and at one of its ends an eyelet provided with an external edge that is designed to interact with one of the aforesaid projections for fixing the thread in a closed-loop operative configuration. Said solution enables a rapid fixing of the suture thread. However, the device according to the patent in question can adversely affect the natural course of cicatrization of the wounds in which it is applied. In fact, said device has a highly irregular outer surface provided with sharp edges throughout its extension, which is likely to cause highly undesirable tissue adhesions to the suture device itself that disturb the process of cicatrization and generate an excessive inflammation in the areas surrounding the lesion.

The object of the present invention is to improve the solution described above by providing a suture thread presenting the characteristics of Claim 1.

The invention will now be described in detail purely by way of non-limiting example, with reference to the annexed plates of drawings, in which:
- Figure 1 illustrates a perspective view of the device according to the invention;
- Figure 2 illustrates a perspective view of the device of Figure 1 in an operative condition;
- Figures 3 to 5 show different embodiments of the device according to the invention in the operative condition;
- Figures 6 to 11 are cross-sectional views of different embodiments of a part of the device according to the invention;
- Figures 12a, 12b, 12c are cross-sectional views of different embodiments of a further part of the device according to the invention;
- Figure 13 is a cross-sectional view of an embodiment of the device according to the invention in the operative condition;
- Figure 14 illustrates a variant embodiment of the device according to the invention;
- Figure 15 is a perspective view of a detail of the device of Figure 14;
- Figure 16 shows a further embodiment of the device according to the invention;
- Figures 17 to 19 are schematic illustrations of three subsequent steps of the application on a lesion of the suture device described herein;
- Figure 20 shows a cross-sectional view of a further embodiment of the device according to the invention in the operative condition.

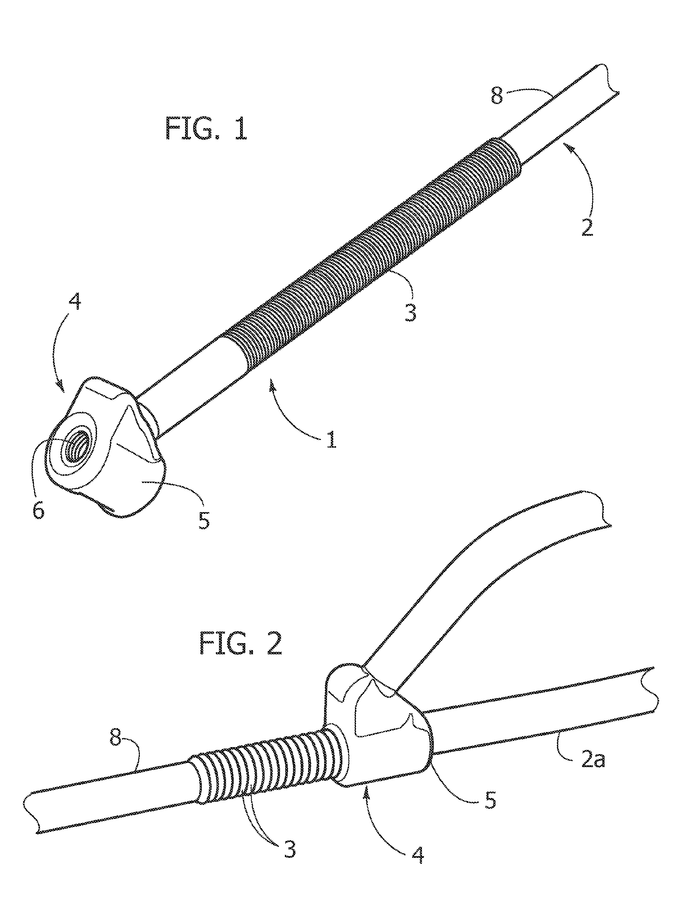

With reference to Figure 1, the reference number 1 designates the surgical suture device according to the invention, which comprises a flexible oblong body 2. The body 2 can be made of a biocompatible polymeric material that can be modelled according to different geometries by means of injection-moulding processes. For example, the body can be made of a biodegradable polymer, such as polylactic acid (poly(D,L-lactide) - PLA) or else poly(D,L-lactide-co-glycolide) (PLGA) copolymers. These polymers are biodegradable in vivo so that within the human body they are reduced, by means of enzymatic or non-enzymatic processes, into biocompatible and non-toxic molecules, which can be readily cleared through the normal metabolic pathways.

Alternatively, the body 2 can be made of any other material already used for the production of conventional suture threads, for example steel, polypropylene, or else polyamide.

The body 2 has a portion, the outer surface of which is provided with a plurality of projections 3 distributed in the direction of extension of the body 2.

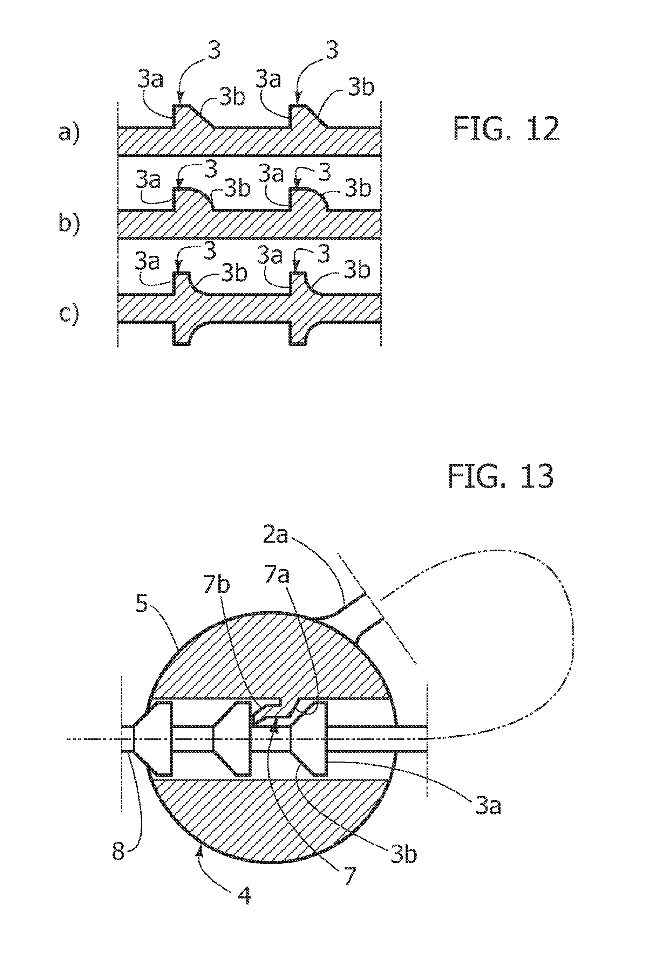

With reference to Figure 12, the projections 3 have a first plane 3a substantially orthogonal to the direction of extension of the body 2 and constituting a plane of contrast, as will emerge from the ensuing description, and a plane 3b that is facing a side opposite to the one facing the plane 3a. The plane 3b has a general ramplike conformation, different embodiments of which are illustrated in Figure 12. In particular, Figure 12a shows a ramp 3b constituted by an inclined plane, Figure 12b represents, instead, a ramp with curvilinear evolution and a concavity facing downwards, and finally Figure 12c shows a ramp with curvilinear evolution and a concavity facing upwards.

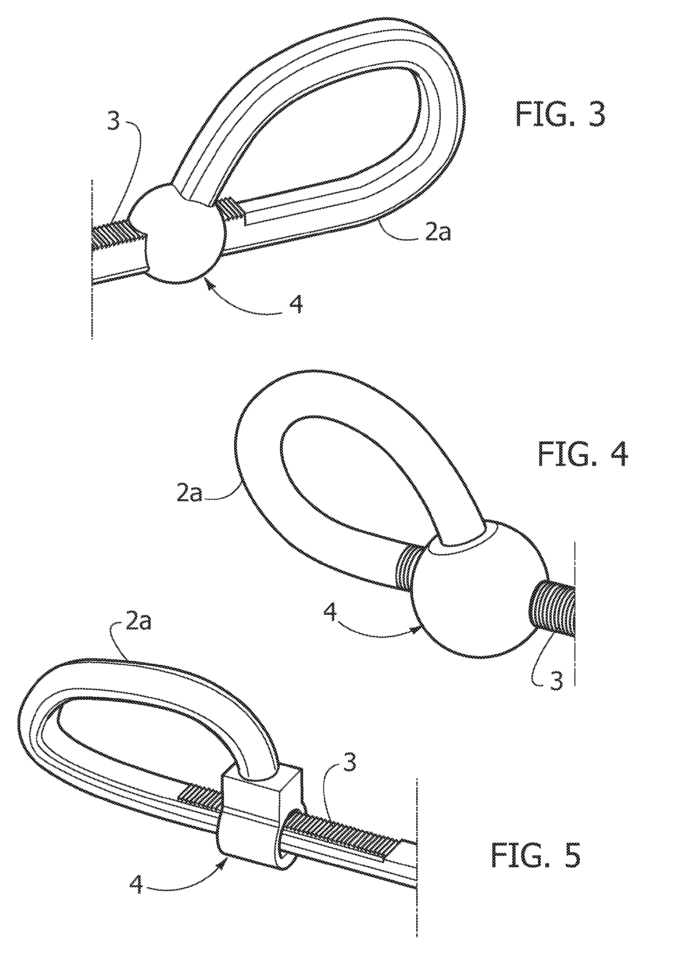

With reference to Figure 1, the body 2 moreover comprises a clamping element 4 set at one end of the body 2. The clamping element 4 is formed by a casing 5 provided with a through opening 6. Figures 2 to 5 show devices of the type described herein, provided with casings of different shapes.

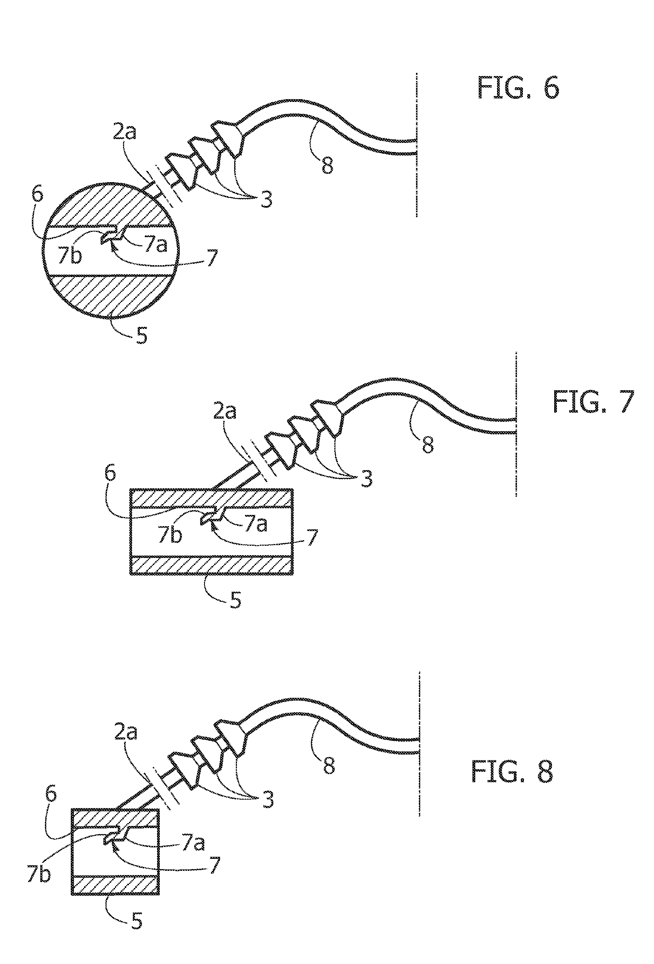

The casing 5 envisages internally an arrest element 7 (illustrated in Figure 6), provided for interacting with the projections 3 described above. The arrest element 7 is preferably formed by a flexible tab that extends from the wall of the opening towards the centre of the latter. With reference to Figure 6, the tab 7 has a cross-sectional profile on a longitudinal plane thereof so that it is flexible if subjected to stress on a side 7a, and substantially rigid, instead, if subjected to stress on the opposite side 7b. The tab 7 can also develop in such a way as to reproduce the profile as described above and, for example illustrated in Figure 6, on the entire perimetral edge of a cross section of the opening 6.

Between the clamping element 4 and the projections 3, the body 2 preferably envisages a smooth outer surface 2a of variable length according to the application, as represented in Figures 2 to 5.

Beyond the projections 3, on the opposite side with respect to the clamping element 4, the body 2 is provided with a further smooth portion 8, the end of which is gripped and manipulated by the surgeon for adjusting the tension of tightening of the device prior to fixing thereof. Said smooth portion is referred to as "thread-tensioner portion".

The smooth portion 8 is of variable length according to the type of operation of suture to be performed. In particular, in surgical interventions supported by endoscopy said portion of thread has a length such as to allow the surgeon to manipulate the device from outside the body of the patient. The end of the portion 8 can moreover be equipped or not with a needle.

The use of the device described herein in an operation of suture envisages tightening the device in a loop configuration, within which the margins of the lesion to be sutured are kept in contact with one another.

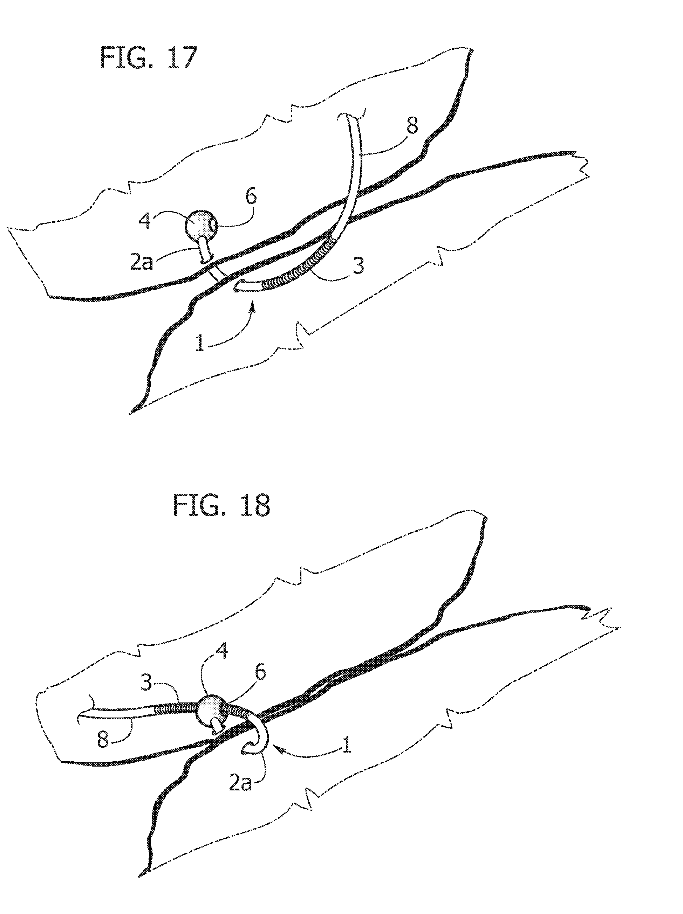

In Figures 17 to 19 are schematic representations of three subsequent steps of an example of application of the suture device described herein on a lesion.

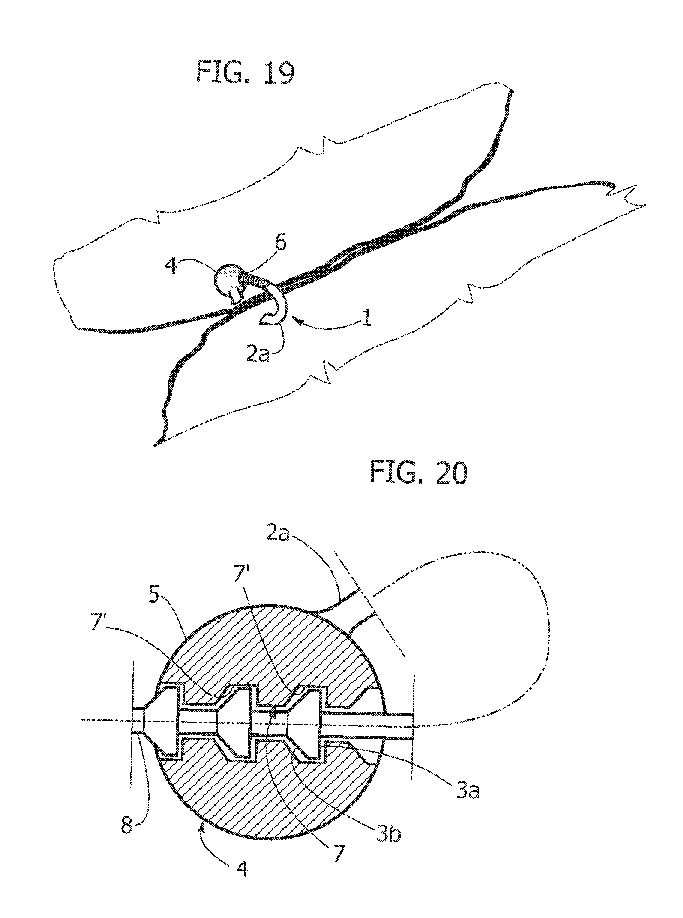

In particular, represented in Figure 17 is the device 1, which has been inserted through the two margins of the lesion in such a way that the portion 2a is at the edges of the two margins themselves. The insertion of the device through the lesion can be performed according to conventional modalities using, for example, a needle provided on its distal end with the thread-tensioner portion 8. Figures 18 and 19 illustrate, instead, the arrangement of the device 1 in the aforementioned tightened condition, where in particular Figure 19 shows the device as finally left operative on the lesion. Said arrangement is reached according to the modalities that will be described in what follows. Furthermore, applications of the device described herein can also envisage the use of accessory elements known in the field of the techniques of conventional suturing, such as, for example, "anchoring" elements fixed on the tissues immediately surrounding the lesion and designed to constitute a firm constraint of the device according to known modalities.

Examples of suture devices according to the invention in the tightened condition are represented in Figures 2 to 5 and in Figure 13. To achieve said configuration, the distal end of the thread-tensioner portion 8 is passed through the opening 6 of the clamping element 4 and pulled until it is obtained that the portions of the loop thus formed press with a required pressure on the tissues enclosed therein. Said operation constitutes the operation of tightening and tensioning of the device.

With reference to Figure 13, the side of insertion of the end of the smooth portion 8 in the opening 6 must be the side for which the planes 3b of the projections 3 face the side 7a of the tab 7 in such a way that, when the thread is pulled in the direction of tightening and tensioning of the device, the projections 3 that impinge upon the tab 7 bend the latter, thus allowing the body 2 to slide through the clamping element 4. The operative condition of the device is instead stably maintained in order to contrast the plane 3a of a projection 3 against the side 7b of the tab 7. Said contrast prevents sliding of the body 2 through the clamping element 4 in the direction of undoing of the loop, opposite to the direction in which the operation of tightening and tensioning is performed.

The step between each projection 3, their total number, and their distance from the clamping element 4 constitute factors chosen in the design stage on the basis of the type of suture operation for which the device is designed.

Furthermore, in order for the surgeon to be able to identify immediately the side of insertion of the end of the portion 8 in the opening 6 of the clamping element, a preferred embodiment of the invention envisages distinctive signs of said side of insertion on the outer surface of the casing 5.

Once the device has been fixed on the lesion, the portion of the body 2 that exits from the clamping element 4, comprising the thread-tensioner portion 8 and possibly some projections 3, is cut away from the looped portion of the device that has been obtained, the device being in the configuration illustrated in Figure 19.

The suture device described herein in the tightened configuration principally exposes substantially regular and smooth surfaces to the surrounding tissues, thus rendering extremely reduced the possibility of there being set up anomalous processes of cicatrization for the reasons described previously. The loop within which the margins of the lesion constituting the operative portion of the device are contained is in fact formed externally by the smooth portion 2a and by the casing 5. The casing 5 has, in this connection, the function of separating from outside the surfaces of fixing of the device constituted by the projections 3 and by the arrest element 7, which are inevitably provided with an extremely irregular conformation that is likely to alter the cicatrization of the tissues, and to present to the interface with the latter a completely smooth surface for the purposes and the advantages mentioned above.

From the foregoing it emerges that the suture device according to the invention enables the suture of a lesion to be performed in a rapid way, at the same time enabling a regular course of cicatrization thereof.

The present applicant has moreover verified that the casing 5 provides a protection of the arrest element 7 and of the projection 3 in mutual engagement, which is able to slow down their process of degradation with respect to that to which the exposed parts of the device are subject, consequently ensuring a stable fastening of the device throughout its entire useful life.

Furthermore, the casing 5 of the clamping element can be made with a conformation such as come into contact with and press on one of the margins of the lesion via a relatively extensive surface so as to distribute the stress exerted on the device over an equally extensive area of the tissues for the purpose of reducing the effect of cutting caused thereon by the device. For example, the casing can present a disklike conformation.

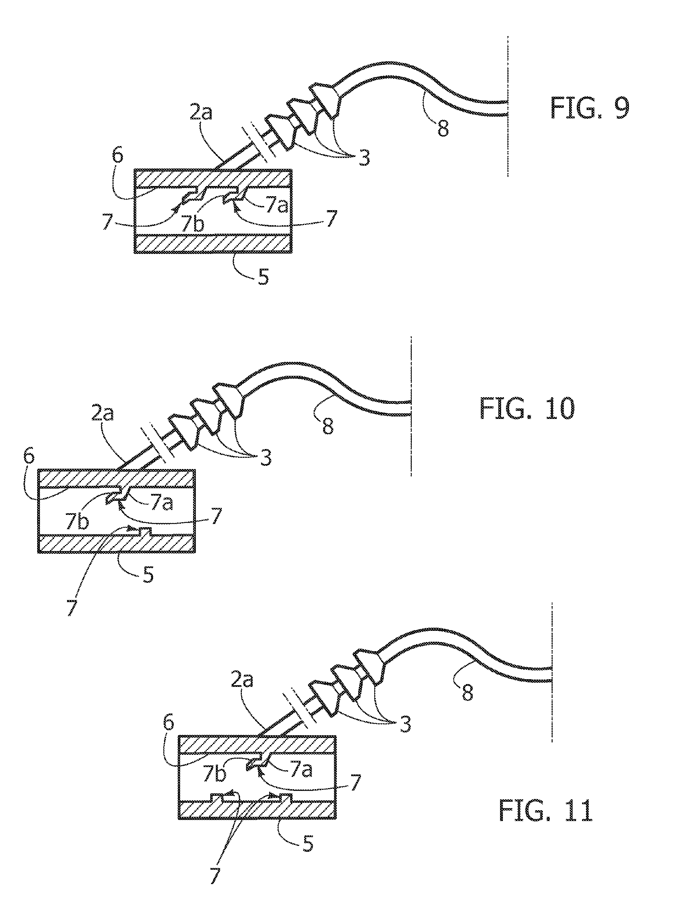

Figures 9 to 11 show embodiments in which the clamping element envisages multiple elements of arrest. Figure 9 shows, by way of example, a clamping element 4 provided with two tabs 7, arranged in succession in the longitudinal direction of the opening 6, which are provided for interacting, respectively, with two different projections 3.

Figure 10 shows, as further example, a clamping element 4 provided with two tabs 7 arranged, respectively, on opposite sides of the internal surface of the opening 6.

Finally, Figure 11 shows a clamping element 4 in which two fixed teeth are provided, arranged respectively at the sides of the tab 7 in a position corresponding to the portion of wall of the opening 6 opposite to the one from which the tab 7 projects.

In the cases where the device described herein is provided with a clamping element 4 as illustrated in Figures 10 and 11, the projections 3 of the body 2 are constituted by circumferential flaps, as illustrated in Figure 12c.

As may be seen in Figure 20, it is likewise possible to provide a clamping element 4, which, instead of envisaging individual elements of arrest of the type described above, has a through opening 6, the internal wall of which is prepared in such a way as to present recesses 7' that are substantially radial and complementary to the projections 3. Surfaces 7b' of said recesses, in contrast with the planes 3a of the projections 3, constitute the arrest 7 designed to fix the tightened condition of the device, preventing any sliding of the body 2 through the clamping element 4 in the direction of undoing of the loop, in a similar way to what has already been described above. In this embodiment of the device, when the thread is pulled in the direction of tightening and tensioning of the device, the projections 3 and the recesses 7' are deformed both elastically for the purpose of enabling sliding of the body 2 through the clamping element 4.

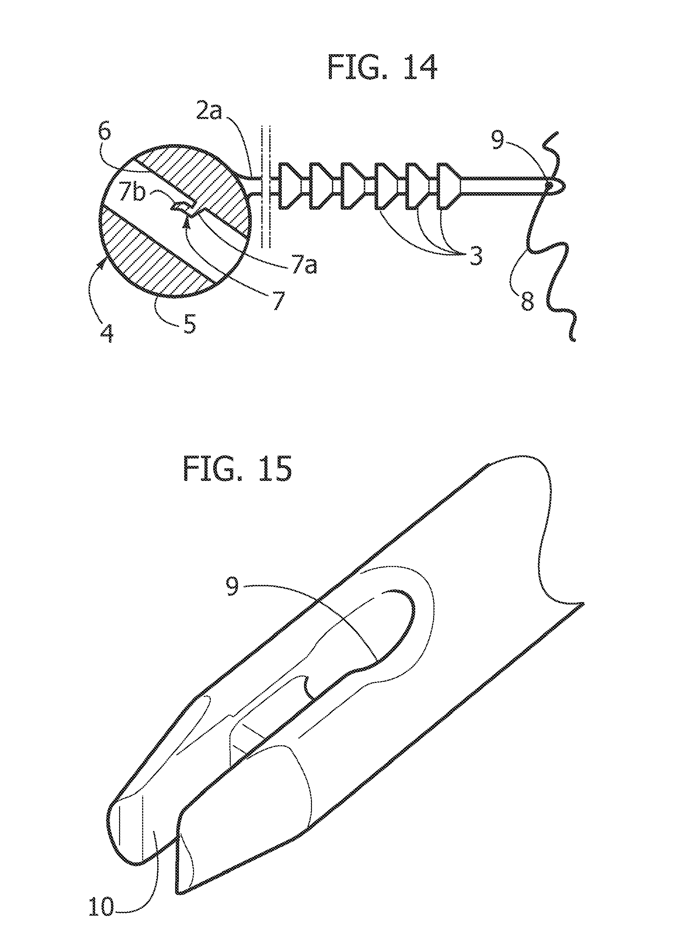

Figure 14 shows an embodiment of the device according to the invention in which the thread-tensioner portion 8 is constituted by an element separate from the body 2. In particular, the body 2 terminates in the proximity of the latter of the projections 3 with an eyelet 9, illustrated in detail in Figure 15. In this case, the thread-tensioner portion is anchored to the eyelet 9 via a knot that can be made already in the step of production of the device or else in the moment of its use. As may be seen once again in Figure 15, beyond the eyelet 9, provided in a position corresponding to the end of the body 2, is a slit 10 designed to receive the knot made with the thread-tensioner portion, designed for anchorage of the latter to the body 2.

Said embodiment has the advantage of enabling use of conventional threads as thread-tensioner elements in such a way that the body 2 is made according to a standardized length, thus considerably simplifying production thereof.

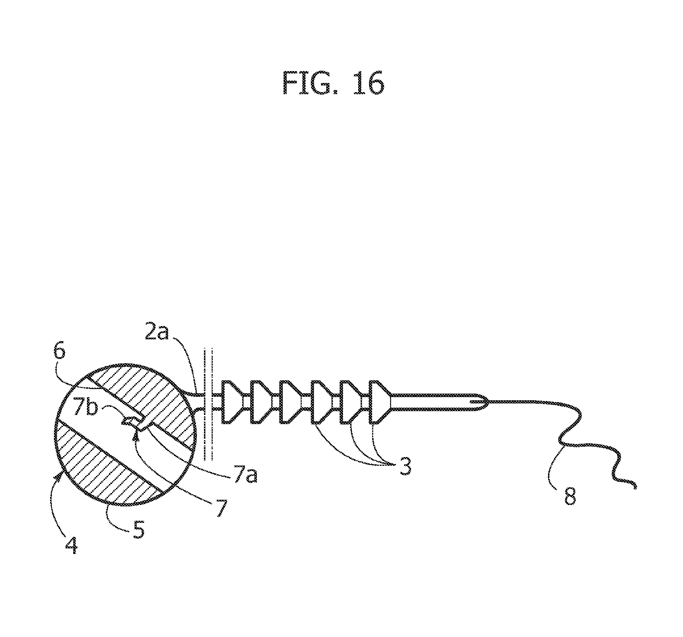

Figure 16 shows a variant of the latter embodiment of the device so that the thread-tensioner portion is connected to the body 2 via an operation of overmoulding or similar technology.

The surgical suture device according to the present invention enables the operations of suture to be carried out more rapidly and without the need for making knots, obtaining, moreover, a mechanical resistance of the suture that is more readily reproducible than and qualitatively superior to the one that can be obtained using techniques of a conventional suture.

Of course, without prejudice to the principle of the invention, the details of construction and the embodiments may vary widely with respect to what is described and illustrated purely by way of example, without thereby departing from the scope of the present invention.

## Claims

1. A surgical suture device comprising a flexible oblong body (2) that can be bent into a loop configuration, in which an engagement portion (3) and a clamping element (4) made in said body are brought into mutual engagement for fixing the loop configuration, said engagement portion presenting an outer surface provided with at least one projection (3), designed to contrast against an arrest element (7) of said clamping element (4), said device being **characterized in that** said clamping element comprises a casing (5) that surrounds said arrest element (7).

2. The surgical suture device according to Claim 1, **characterized in that** said casing (5) has a substantially smooth outer surface.

3. The surgical suture device according to Claim 1, **characterized in that** a portion (2a) of said body between said clamping element (4) and said engagement portion (3) has a substantially smooth outer surface.

4. The surgical suture device according to Claim 1, **characterized in that** said arrest element (7) is formed by a flexible tab.

5. The surgical suture device according to Claim 4, **characterized in that** said casing (5) has a through opening (6), from the walls of which there extends centrally said tab (7), said tab (7) allowing said engagement portion (3) to move within said opening according to just one direction of insertion.

6. The surgical suture device according to Claim 5, **characterized in that** said tab (7) has a cross-sectional profile on a longitudinal plane thereof so that it is flexible if subjected to stress on one side (7a), and rigid if subjected to stress on the opposite side (7b).

7. The surgical suture device according to Claim 1, **characterized in that** a thread-tensioner portion extends subsequent to said engagement portion and opposite to said clamping element, which is provided for remote manoeuvring of the device from the lesion to be sutured.

8. The surgical suture device according to Claim 7, **characterized in that** said thread-tensioner portion (8) is made on said body (2).

9. The surgical suture device according to Claim 7, **characterized in that** said body (2) comprises an eyelet (9), to which said thread-tensioner portion (8) can be connected.

10. The surgical suture device according to Claim 7, **characterized in that** said thread-tensioner portion (8) is overmoulded on said body (2) or is connected thereto in a similar way.

11. The surgical suture device according to Claim 1, **characterized in that** said engagement portion has a plurality of projections.

12. The surgical suture device according to Claim 11, **characterized in that** said projections have shapes comprised in the group including: wedge shape, sawtooth shape, conical shape, and spiral shape.

13. The surgical suture device according to Claim 7, **characterized in that** said thread-tensioner portion is provided with a needle.

14. The surgical suture device according to Claim 1, **characterized in that** said body is made of biodegradable polymeric material, such as polylactic acid (poly(D,L-lactide) - PLA) or else poly(D,L-lactide-co-glycolide) (PLGA) copolymers.

15. The surgical suture device according to Claim 5, **characterized in that** the casing (5) has distinctive signs of said direction of insertion.

16. The surgical suture device according to Claim 1, **characterized in that** said arrest element is constituted by recesses (7') made on an internal wall of said casing (5).
